# EUROPEAN PATENT APPLICATION

(11) **EP 4 455 260 A1**
(43) Date of publication of application: **30.10.2024**
(21) Application number: 22910244.7
(22) Date of filing: 26.12.2022
(51) Int. Cl.: C12M 1/34, C12Q 1/6869

(54) **BIOCHIP FOR SPATIAL TRANSCRIPTOMIC ANALYSIS, AND PREPARATION METHOD AND APPLICATION THEREOF**

(30) Priority: 24.12.2021 CN 202111601551; 25.06.2022 CN 202210728404
(71) Applicant: Intelligent Healthcare Technology Limited, Kowloon, Hong Kong (HK)
(72) Inventor: ZHAO, Haifeng, Hong Kong (CN)
(74) Representative: Laine IP Oy
(86) International application number: PCT/CN2022/142132
(87) International publication number: WO 2023/116938

(57) **Abstract**

A chip for analyzing nucleic acid information of a biological sample, and a method of preparing the chip, the chip being suitable for analyzing spatial transcriptomic information of a biological tissue sample. Additionally provided is a method for analyzing spatial transcriptomic information of a biological tissue sample. The method and device can effectively obtain nucleic acid expression information in cells of a tissue sample, including spatial omics information.

## Description

This application claims priority to Chinese patent application No. 202111601551.X, filed on December 24, 2021, entitled "Biochip for spatial transcriptomics analysis, and preparation method thereof and application thereof", and Chinese patent application No. 202210728404.7, filed on June 25, 2022, entitled "Biochip for spatial transcriptomics analysis, and preparation method thereof and application thereof", the entire contents of which are incorporated herein by reference.

### Technical Field

The present invention relates to the fields of biology and medical devices. Specifically, the present invention relates to a method of preparing a microarray for analyzing nucleic acid information of cells of a biological sample, said microarray being suitable for analyzing spatial transcriptomics information of a biological tissue sample.

### Background

The analysis of cellular organization and expression patterns of biological tissues is a milestone in biomedical research and diagnostics. Using a variety of staining techniques, developments in the field of cellular histology studies have led to the possibility of studying protein distribution by immunohistochemistry and gene expression by in situ hybridization.

New studies hope to characterize the transcriptome and/or genomic variation in tissues while preserving spatial information about the tissues.

The prior art employs microarrays for analyzing nucleic acid information of cells of a biological sample, in which methods for forming probes for recognizing and binding nucleic acids of a biological sample on a substrate such as a slide and microarray products carrying these probes are provided.

There is still a need in the field for preparation processes and products that can more easily and efficiently form probes on chip substrates capable of binding target DNA or RNA molecules in the large-scale production of chips used to analyze nucleic acid information of cells of biological samples, thereby obtaining chips of better quality, as well as making the yield more stable.

### Summary

The present invention provides a method of preparing a microchip in a big scale for analyzing nucleic acid information of cells of a biological tissue sample, in particular suitable for analyzing spatial transcriptomics information.

Specifically, the present invention provides an improved method for preparing a biochip having an array, comprising the steps of:
Step 1. providing a chip;
Step 2. fixing a chip surface linker nucleic acid on the chip surface, for example on the whole surface of the chip;
Step 3. applying a first set of barcode nucleic acids on the surface of the chip through a plurality of microfluidic channels set in parallel to form a plurality of first barcode strips in a first direction under the condition of enabling a joining reaction between said chip surface linker nucleic acid and the first barcode nucleic acids, wherein said first set of barcode nucleic acids comprise a plurality of first barcode nucleic acids having different barcode sequences, each said first barcode strip contains one kind of first barcode nucleic acid, and each said kind of first barcode nucleic acid fixed in each first barcode strip has a different barcode sequence;
Step 4. applying a second set of barcode nucleic acids in a second direction on the chip surface through a plurality of microfluidic channels set in parallel to form a plurality of second barcode strips, said second set of barcode nucleic acids comprise a plurality of second barcode nucleic acids having different barcode sequences, each said second barcode strip contains one kind of second barcode nucleic acid, and each said kind of second barcode nucleic acid contained in each second barcode strip has a different barcode sequence;
Step 5. under conditions that cause the first barcode nucleic acid and the second barcode nucleic acid to undergo a joining reaction, joining the second barcode nucleic acid to the first barcode nucleic acid at a place on the surface of the chip where said plurality of first barcode strips intersect said plurality of second barcode strips to generate probes, said places of the probes (i.e., the place on the surface of the chip where said plurality of first barcode strips intersect said plurality of second barcode strips) forming arrays of dots, each dot containing a probe with a sequence different from each other.

In one of the aspects of the present invention, in aforementioned method, said first set of barcode nucleic acids or second set of barcode nucleic acids are delivered and fixed to the chip surface using a microfluidic device having a plurality of microfluidic channels set in parallel, wherein the side of said microfluidic channel in contact with the chip surface is set to allow passage of solution or nucleic acids in solution.

In one of the aspects of the present invention, in aforementioned method, one of the first set of barcode nucleic acids or the second set of barcode nucleic acids containing different barcode sequences is added into each microfluidic channel of said microfluidic device.

In one of the aspects of the present invention, the chip surface linker nucleic acid is immobilized on the chip surface via a chemical bonding means. The chemical bonding means includes any chemical group reactions, for example, amino-aldehyde group reaction and the like, or covalent cross-linking. The surface of the chip may be encapsulated by a surface chemical reaction with an amino group, an aldehyde group, an epoxy group, an isothiocyanate group, a sulfhydryl group, a silane and other reactive groups and the like. The end (usually the 5' end) of said chip surface linker nucleic acid connected to the chip surface then has a group that forms a chemical bond with the encapsulated reactive group.

In yet another one of the aspects of the present invention, said chip surface linker nucleic acid may also be immobilized on the chip surface by physical adsorption, e.g., by hydrophobic interaction, electrostatic attraction and the like. This can be done, for example, by modifying the chip surface with poly-L-lysine (PLL) or by treating it with a surfactant, for example.

In one of the aspects of the present invention, in aforementioned method, said chip surface linker nucleic acid has a linking fragment at the 3' end. In one of the aspects of the present invention, said first barcode nucleic acid has a linking fragment at the 5' end for linking to the chip surface linker nucleic acid via said first single-stranded linking nucleic acid, said linking fragment at the 3' end of chip surface linker nucleic acid and said linking fragment at the 5' end of the first barcode nucleic acid are each complementary to a sequence at each end of said first single-stranded linking nucleic acid.

In one of the aspects of the present invention, said chip surface linker nucleic acid has about 10-50 nucleotides, preferably having less than 30 nucleotides, for example less than 25 nucleotides.

In one of the aspects of the present invention, in aforementioned method, said first barcode nucleic acid of said first set of barcode nucleic acids comprises a first barcode fragment. In one of the aspects of the present invention, in aforementioned method, said first barcode nucleic acid has a first linking fragment at the 3' end for linking to the second barcode nucleic acid and a linking fragment at the 5' end for linking to said chip surface linker nucleic acid.

In one of the aspects of the present invention, in aforementioned method, said second barcode nucleic acid in said second set of barcode nucleic acids comprises a capture fragment at the 3' end for recognizing and binding a target nucleic acid in the biological sample (e.g., a fragment for recognizing and binding mRNA or cDNA, e.g., a poly-T sequence) and a second barcode fragment.

In one of the aspects of the present invention, in aforementioned method, said second barcoded nucleic acid in said second set of barcoded nucleic acids further has a unique molecular identifier (UMI).

In one of the aspects of the present invention, in aforementioned method, said first barcode nucleic acid has a first linking fragment at the 3' end for linking to the second barcode nucleic acid via a second single-stranded linking nucleic acid, and said second barcode nucleic acid has a second linking fragment at the 5' end for linking to the first barcode nucleic acid via said second single-stranded linking nucleic acid, said first linking fragment and second linking fragment are each complementary to a sequence at each end of said second single-stranded linking nucleic acid.

In one of the aspects of the present invention, in aforementioned method, the probe formed in step 5 comprises a capture fragment at the 3' end for identifying and binding a target nucleic acid in the biological sample, optionally a unique molecular identifier (UMI), a second barcode fragment and a first barcode fragment, and chip surface linker nucleic acid at the 5' end. In one of the aspects of the present invention, said chip surface linker nucleic acid further has a primer fragment at the 5' end for use in an amplification reaction.

In one of the aspects of the present invention, in aforementioned method, the sequence of the first barcode fragment of each of said first barcode nucleic acids of said first set of barcode nucleic acids is designated, and/or the sequence of the second barcode fragment of each of said second barcode nucleic acids of said second set of barcode nucleic acids is designated.

In one of the aspects of the present invention, in aforementioned method, the sequence of the barcode fragment of each of said first barcode nucleic acids of said first set of barcode nucleic acids and the sequence of the barcode fragment of each of said second barcode nucleic acids of said second set of barcode nucleic acids is designated.

In one of the aspects of the present invention, in aforementioned method, the chip surface linker nucleic acid is added to the surface of the chip at a concentration of about 0.1-100µM/L, for example, about 1-20µM/L.

In one of the aspects of the present invention, in aforementioned method, the concentration of nucleic acid in the flow channel in step 3 is about 0.1-100µM, for example, about 1-20µM.

In one of the aspects of the present invention, in aforementioned method, the concentration of nucleic acid in the flow channel in step 4 is about 0.1-100µM, for example, about 1-20µM.

In one of the aspects of the present invention, the width of each channel of said microfluidic channels set in parallel in step 3 and step 4 is about 2-200µm, preferably about 5-50µm, most preferably about 5-25µm, for example about 5µm, 10µm or 50µm.

In one of the aspects of the present invention, the spacing between each adjacent microfluidic channel of the microfluidic channels set in parallel is about 5-400µm, preferably about 10-100µm, most preferably about 10-50µm, such as about 20µm, 50µm or 100µm.

In one of the aspects of the present invention, in array dots of the chip obtained by the aforementioned method, the probes have a density of about 10³-10⁵ probes/µm².

In one of the aspects of the present invention, in array dots of the chip obtained by the aforementioned method, the probes have a homogeneity deviation of less than 20%, preferably less than 10%, more preferably less than 6%.

In one of the aspects of the present invention, in array dots of the chip obtained by the aforementioned method, the probes have a deviation in dimensional homogeneity of less than 10%, preferably less than 5%, more preferably less than 2%.

The present invention also provides a biochip for analyzing nucleic acid information of a biological sample. In one aspect of the present invention, said biochip is prepared by the method aforementioned. In one aspect of the present invention, said biochip has a surface having probes forming an array thereon, said array comprising orthogonal rows and columns of probes, the probe in each spot of said array having a different code sequence, which indicated the spacious information of the probe. In one aspect of the present invention, said probes comprise a first barcode and a second barcode, each row of probes of said array of probes has the same first barcode and each column of probes has the same second barcode; each row of probes has a first barcode different from the other row and each column of probes has a second barcode different from the other column. In one aspect of the present invention, there are a chip surface linker nucleic acid on the whole surface of said biochip. In one aspect of the present invention, the 5' end of each probe in the array is the chip surface linker nucleic acid. In one aspect of the present invention, each probe in said probe array comprises, from the 5' end to the 3' end, said chip surface linker nucleic acid, a first barcode, a second barcode, and a capture fragment for recognizing and binding to a target nucleic acid in a biological sample. In one aspect of the present invention, each probe in said probe array comprises a primer fragment at the 5' end for use in an amplification reaction. In one aspect of the present invention, each probe in said probe array further has a unique molecular identifier (UMI).

In one aspect of the present invention, in array dots of the chip obtained, the probes have a density of about 10³-10⁵ probes/µm². In one aspect of the present invention, in array dots of the chip obtained, the probes have a homogeneity deviation of less than 20%, preferably less than 10%, more preferably less than 6%. In one aspect of the present invention, in array dots of the chip obtained, the probes have a deviation in dimensional homogeneity of less than 10%, preferably less than 5%, more preferably less than 2%.

The microarrays provided by the present invention and the microarrays obtained by the preparation of the present invention can be used for the analysis of tissue samples, in particular tissue thin sections, for the analysis of molecules contained in cells, including the analysis of nucleic acids and proteins, for example by PCR, mass spectrometry, next-generation sequencing, or ELISA, to obtain expression and spatial information thereof.

The present invention provides a method for analyzing spatial transcriptomic information of a biological tissue sample by using a biochip having an array. Said method comprises contacting the array of the biochip with the tissue sample, the probes on the array recognizing and binding nucleic acids, in particular mRNA, of cells in the tissue. In one aspect of the present invention, the nucleic acids leave the cells of the tissue and bind with the probes on said biochip. In one aspect of the present invention, when said tissue is a fixed and embedded tissue, the nucleic acids are released from the cells by permeabilizing said tissue.

In yet another aspect of the present invention, the method further comprising performing a reverse transcription reaction.

In yet another one of the aspects of the present invention, said method further comprises: isolating and purifying the cDNA after collection of the tissue/cells, e.g., after separating the tissue from the microarray, dividing the tissue/cells as well as isolating and purifying the nucleic acids in a container.

In yet another aspect of the present invention, the method further comprising amplifying said cDNA molecule.

In yet another aspect of the present invention, the method further comprising performing library building/sequencing of the amplified obtained nucleic acids.

In one of the aspects of the present invention, said biological sample is a tissue sample from a subject, for example a surgically excised tissue sample, preferably a thin section of tissue obtained by microtome processing. In one aspect of the present invention, said tissue sample is fixed and embedded (e.g. embedded in paraffin), and attached to a support such as a slide. In one of the aspects of the present invention, the nucleic acids of the cells in the immobilized tissue leave the tissue after permeabilization to bind to the probe on the chip.

In one aspect of the present invention, said tissue thin section may be subjected to morphological analysis and/or histological analysis, which is performed by H&E staining, IHC staining, ISH staining, or FISH staining.

In one aspect of the present invention, the analysis of the one or more biomolecules is performed by PCR, mass spectrometry, next generation sequencing, or ELISA.

In one aspect of the present invention, the subject is selected from an animal, a farm animal, a pet, a human subject.

In one aspect of the present invention, the analyte further comprises one or more of a non-human cell, a human cell, an unnatural protein, a nucleic acid, or a small molecule, a dye, a virus, a bacterium, a parasite, a protozoan, or a chemical substance. Small molecules include semi-antigens, peptide tags, protein tags, fluorescent tags, nucleic acid tags, and combinations thereof.

In one aspect of the present invention, said microarray may be used to analyze quantitative and/or qualitative data for a marker in a sample. The marker comprises DNA, proteins, RNA, lipids, organelles, metabolites, or cells. The markers include genomic polymorphisms, pharmacogenomic single nucleotide polymorphisms (SNPs), genomic SNPs, somatic polymorphisms, and differential expression of proteins, lipids, and/or organelles. The markers include individual nucleotide positions; intragenic regions or intergenic regions; exons or introns, or fragments thereof; coding or non-coding regions; promoters, enhancers, 5 ' untranslated regions (5 'UTRs), or 3 'untranslated regions (3 'UTRs), or fragments thereof; cDNAs or fragments thereof; SNPs; somatic mutations, germline mutations, or both; point mutations or single mutations; deletion mutation; in-frame deletion, in-gene deletion, whole gene deletion; insertion mutation; in-gene insertion; inversion mutation; intrachromosomal inversion; linked mutation; linked insertion mutation; inverted repeat mutation; tandem duplication; intrachromosomal tandem duplication; translocation; chromosomal translocation, non-mutual translocation; rearrangement; genome rearrangement; rearrangement of one or more introns, or fragments thereof; rearrangement of an intron; a 5 '- or 3 ' -UTR, or a combination thereof. Wherein the marker may be measured by single cell sequencing, single nucleus sequencing, flow cytometry, immunohistochemical staining, hematoxylin and eosin staining, whole genome sequencing, high throughput sequencing, mass spectrometry, DNA microarrays, or combinations thereof.

The microchip of the present invention may be used to analyze a tissue sample. The tissue sample comprises a sample selected from the group consisting of: one or more premalignant or malignant cells, cells from solid tumors, soft tissue tumors or metastases, tissue or cells from surgical margins, histologically normal tissue, one or more circulating tumor cells (CTCs), normal adjacent tissue (NAT), blood samples from the same subject suffering from, or at risk of, developing a tumor, or FFPE samples.

### Brief description of the drawings

In order to more clearly illustrate the technical solutions in the embodiments or prior art of the present invention, the following will be a brief introduction to the accompanying drawings that need to be used in the description of the embodiments or prior art.
FIG. 1 shows a schematic diagram of the steps of a method for preparing a chip provided by the present invention.
FIG. 2 shows an exemplary embodiment of the method of the present invention using an apparatus having a plurality of microfluidic channels disposed in parallel.
FIG. 3 shows a diagram of the observation of a probe dot fluorescence signal of a biochip provided by the present invention.
FIG. 4 shows a graph of detection of a dot array of a biochip provided by the present invention based on the probe fluorescence signal. FIGS. 4A and 4B show measurements of the size and fluorescence intensity of the dot array, respectively.
FIG. 5 shows a tissue HE staining graph for spatial transcriptomics analysis of mouse brain tissue by the biochip provided by the present invention.
FIG. 6 shows an observation of the preparation process of the biochip provided by the present invention on its barcode nucleic acid ligation reaction.
FIG. 7A is a graph of the number of genes detectable by spatial transcriptomics analysis of mouse brain tissue via the biochip provided by the present invention.
FIG. 7B shows a graph of the number of UMIs detectable by spatial transcriptomics analysis of mouse brain tissue by the biochip provided by the present invention.
FIG. 7C is a graph of the distribution of the actual number of genes detected versus the number of captured transcripts (UMI) by spatial transcriptomics analysis of mouse brain tissue via the biochip provided by the present invention.
FIG. 8 shows a graph of the results of gene clustering analysis of spatial transcriptomics analysis of mouse brain tissue by the biochip provided by the present invention.

### Examples

In order to make the objects, technical solutions and advantages of the embodiments of the present invention clearer, the technical solutions in the embodiments of the present invention will be described clearly and completely in the following in conjunction with the accompanying drawings in the embodiments of the present invention, and it is clear that the described embodiments are a part of the embodiments of the present invention and not all of the embodiments. Based on the embodiments in the present invention, all other embodiments obtained by a person of ordinary skill in the art without making creative labor fall within the zone of protection of the present invention.

### Example 1

The present invention provides a method for preparing a biochip suitable for analyzing nucleic acid information of cells of a biological sample. More specifically, the present invention provides a method for preparing a biochip having an array. In one aspect of the present invention, the chip provided by the present invention is adapted to analyze spatial transcriptomics information of a biological tissue sample.

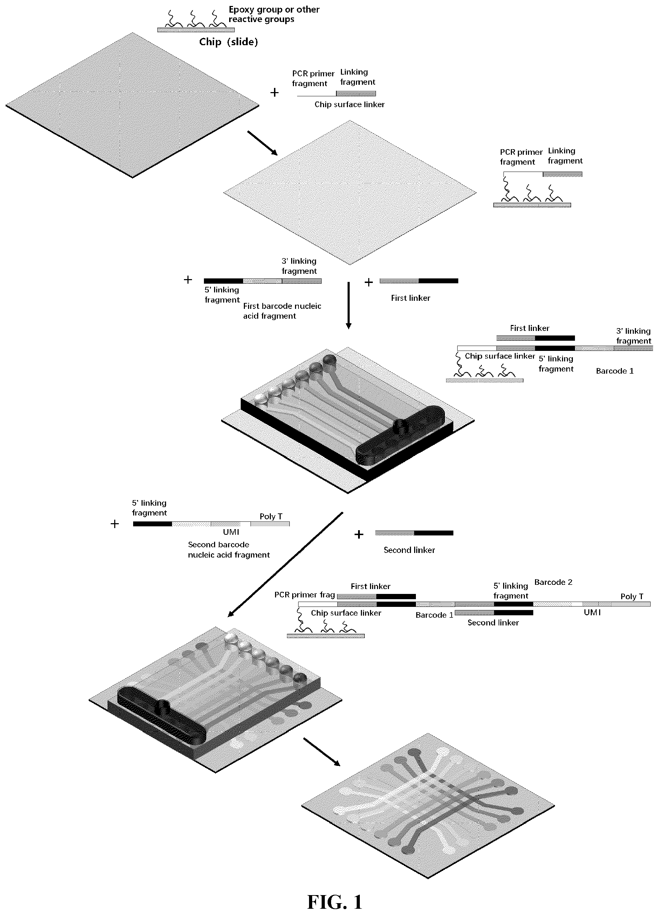

FIG. 1 shows a flow diagram of an exemplary method for preparing a biochip having an array provided by the present invention.

As shown in FIG. 1, the described method mainly comprises the following steps:
Step 1. providing a chip, or named substrate; in order to facilitate the next step of immobilizing a chip surface linker nucleic acid on the chip surface, coating the chip surface via a chemical bonding means, which includes any chemical group reactions, for example, amino-aldehyde group reaction and the like, or covalent cross-linking.
Step 2. fixing a chip surface linker nucleic acid on the chip surface, for example on the whole surface of the chip. The chip surface linker nucleic acid has a linking fragment at the 3' end. In one of the aspects of the present invention, and has a primer fragment at the 5' end for use in an amplification reaction.
Step 3. applying a first set of barcode nucleic acids on the surface of the chip through a plurality of microfluidic channels set in parallel to form a plurality of first barcode strips in a first direction under the condition of enabling a joining reaction between said chip surface linker nucleic acid and the first barcode nucleic acids, wherein said first set of barcode nucleic acids comprise a plurality of first barcode nucleic acids having different barcode sequences, each said first barcode strip contains one kind of first barcode nucleic acid, and each said kind of first barcode nucleic acid fixed in each first barcode strip has a different barcode sequence.

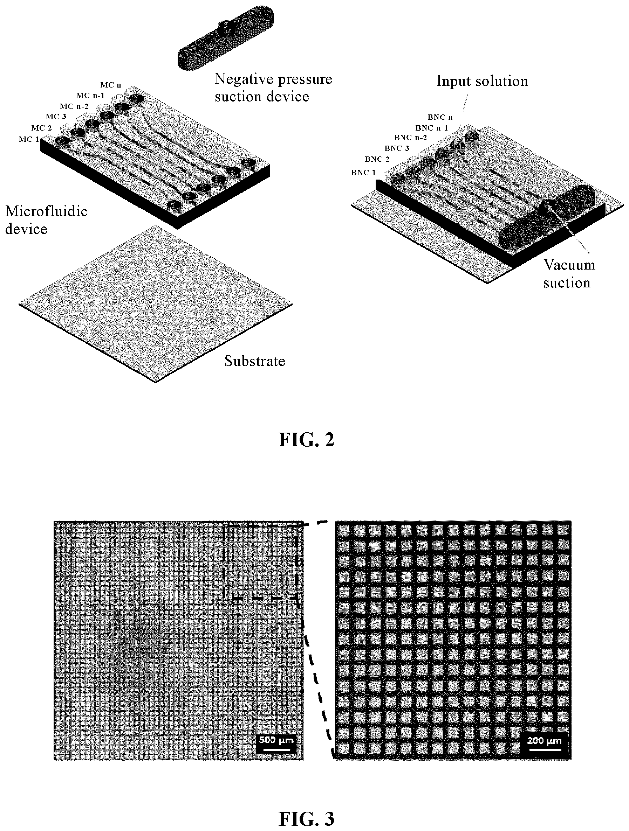

FIG. 2 shows an exemplary embodiment of adding a plurality of barcode nucleic acids to a chip surface by a plurality of microfluidic channels disposed in parallel, joining reaction happens between the plurality of barcode nucleic acids and the chip surface linker nucleic acid, the plurality of barcode nucleic acids are immobilized on the chip surface. The chip is shown at the bottom of the left panel of FIG. 2. The middle of the left panel of FIG. 2 shows a microfluidic device having a plurality of parallelly disposed microfluidic channels, i.e., microfluidic channel (MC) 1-n in FIG. 2, wherein the side of the microfluidic channel in contact with the surface of the chip, i.e., the bottom of the microfluidic channel as illustrated, allows the passage of solution and nucleic acids in solution. For example, the side of said microfluidic channel in contact with the chip surface does not have a microfluidic channel wall. The microfluidic device is placed over the surface of the chip in a first direction, and then a specified solution, such as a solution containing a barcode nucleic acid, is passed within the microfluidic channel. The upper left panel of FIG. 2 shows an exemplary device for assisting in the passage of the solution, such as a vacuum suction device utilizing negative pressure, which may be provided at the outlet of the microfluidic channel. The right panel of FIG. 2 shows the addition of barcoded nucleic acids containing different barcode sequences, i.e., barcoded nucleic acids (BNC) 1 - n in FIG. 2, through the inlet in each of the microfluidic channels. In one aspect of the present invention, the barcode sequence of said barcode nucleic acid passed through the inlet in each microfluidic channel has a known or specified nucleotide sequence.

As shown in FIG.1, the first barcode nucleic acid has a linking fragment at the 5' end for linking to the chip surface linker nucleic acid via a single-stranded linking nucleic acid (the first linker), a linking fragment at the 3' end of chip surface linker nucleic acid and said linking fragment at the 5' end of the first barcode nucleic acid are each complementary to a sequence at each end of said first single-stranded linking nucleic acid.

Step 4. removing the microfluidic channel of Step 3, a second set of barcode nucleic acids is passed through another set of a plurality of microfluidic channels disposed in parallel, in contact with the first barcode strips at the surface of the chip along a second direction that is different from the direction of the first barcode band (typically perpendicular to the first direction), forming a plurality of second barcode strips, wherein the second set of barcode nucleic acids comprise a plurality of second barcode nucleic acids and each of which has a different barcode sequence;and the second barcode nucleic acid immobilized on each of the second barcode strips has a different second barcode sequence.

The illustrated example second barcode nucleic acid comprises a poly-T sequence at the 3' end that recognizes and binds to the mRNA, a unique molecular identifier (UMI), and a second barcode fragment.

In the illustrated example, the 3' end of the first barcode nucleic acid has a first linker fragment for linking to the second barcode nucleic acid via a single-stranded linker nucleic acid (second linker), the 5' end of said second barcode nucleic acid has a second linker fragment for linking to the first barcode nucleic acid via said second linker, and said first linker fragment and the second linker fragment are each complementary to a sequence at each end of said second single-stranded linking nucleic acid. Under conditions that enable the first barcode nucleic acid and the second barcode nucleic acid to undergo a ligating reaction, ligating the second barcode nucleic acid to the first barcode nucleic acid on the surface of the chip where said plurality of first barcode strips produce a crossover with said plurality of second barcode strips, forming a probe.

Step 5. removing the microfluidic channel from step 4 to obtain a biochip having a probe array on the surface. Each array dot on said probe array corresponds to a location where said one of said plurality of first barcode bands forms a crossover with one of said plurality of second barcode strips. Each array dot has a probe molecule comprising a first barcode sequence and a second barcode sequence. Each array dot has a probe molecule comprising a different combination of the first barcode sequence and the second barcode sequence. In one aspect of the present invention, the first barcode sequence and the second barcode sequence of the barcoded nucleic acid passed through each of said microfluidic channels is known or specified, whereby the combination of the first barcode sequence and the second barcode sequence of the probe molecule in each array dot is also known. The spatial position of the probe molecules of each array dot in the array on the chip surface can thus be known from the first barcode sequence and the second barcode sequence of the probe molecules of the respective array dot.

In Step 1, a microchip (or "microarray") typically refers to a solid substrate on which chemical, biological, biophysical or biochemical processes may be implemented. The chip may have microstructures or microscale structures such as channels and pore wells, electrode elements, electromagnetic elements, etc. facilitating the chemical, biological, biophysical or biochemical processes occurring on the chip. The surface of the chip may be flat or uneven. Chips with uneven surfaces may include channels or wells constructed on the surface.

The microchip may be made of any suitable material, and exemplary types of chip materials include glass, modified glass, functionalized glass, inorganic glass, microspheres (including inert and/or magnetic particles), plastics, polysaccharides, nylon, nitrocellulose, ceramics, resins, silicon dioxide, silica-based materials, carbon, optical fibers or fiber optic bundles, a wide variety of polymers in addition to the exemplary materials described above, and porous microtitre plates. plates. Specific types of exemplary plastics include acrylics, polystyrene, copolymers of styrene and other materials, polypropylene, polyethylene, polybutylene, polyurethanes, and Teflon^{™} Specific types of exemplary silica-based materials include various forms of silicon and modified silicon. Deposition of biopolymers (including nucleic acids, peptides, and/or other polymers) is typically required on the chip surface.

In the present invention, said chip surface obtained by preparation has probes (or capture probes) on the array. A probe is a single-stranded nucleotide molecule that can gene-specifically or target-specifically recognize and bind to a target nucleic acid, e.g., a nucleic acid from a tissue sample, and that has a specific nucleotide sequence, i.e., one that can selectively anneal to the targeted nucleic acid, usually a complementary nucleotide sequence. Examples of analytes in tissue samples include genomic DNA, methylated DNA, specific methylated DNA sequences, messenger RNA (mRNA), poly A mRNA, mitochondrial DNA, viral RNA, micro RNA, in situ-synthesized PCR products, RNA/DNA heterodimers, lipids, carbohydrates, proteins. The capture probe may be a gene-specific capture probe which hybridizes, for example, to an mRNA or cDNA specifically targeted in the sample.

In the present invention, said probe has a barcode sequence for use in applications where subsequent high-throughput next-generation sequencing (NGS) or synthetic sequencing (SBS) is performed for analysis, for example in performing large-throughput sequencing analyses. In these sequences, barcode sequences are employed to mark and identify the origin of the nucleic acids of the nucleic acid sequences obtained by sequencing. Barcode molecules are used to barcode nucleic acid molecules (e.g., RNA molecules) from biological particles (e.g., cells) to generate sequencing libraries, and said sequencing libraries are subsequently sequenced to generate a plurality of sequencing read lengths. Some or all of the plurality of sequencing read lengths include barcode sequences. In these sequencing applications, cellular nucleic acids are typically amplified until the barcoded overlapping fragments in the object constitute at least 1X coverage, at least 2X, at least 3X, at least 4X, at least 5X, at least 10X, at least 20X, at least 40X, or higher coverage of a particular portion or all of the cellular genome. Once barcoded fragments are generated, they can be sequenced directly on a suitable sequencing system, such as an Illumina system. The presence of identical barcodes on multiple sequences can provide information about the origin of the sequence.

In the present invention, two barcode sequences are contained in a probe. The two barcode sequences can help to determine the position of the probe in the array on the surface of the chip (determining the position in the X dimension and the Y dimension, respectively), and thus also function as a position label. The barcode sequence on the probe can correspond to an array dot in the array on the chip, and can also indicate the position of a cell, including a single cell, in that tissue sample. Examples of other molecules that may be coupled to a nucleic acid tag include antibodies, antigen-binding structural domains, proteins, peptides, receptors, semi-antigens, and the like.

In the present invention, said probe further comprises one or more unique molecular identifiers (UMIs). The unique molecular identifier is a contiguous nucleic acid fragment or two or more non-contiguous nucleic acid fragments that act as a marker or identifier for a specific analyte or a capture probe that binds to a specific analyte. The UMI is a nucleic acid sequence that does not essentially hybridize with analyte nucleic acid molecules in the biological sample. The UMI may be a nucleic acid sequence comprising from about 6 to about 20 or more nucleotides within the sequence of the capture probe.

In step 2 of the method of present invention, fix (or `immobilization') of chip surface linker nucleic acids to the chip may be performed using various methods known in the art. Immobilization of the nucleic acids refers to direct or indirect attachment to the chip by covalent or non-covalent bonding. In one aspect of the present invention, immobilization refers to remaining attached to the chip during reactions such as those requiring nucleic acid amplification and/or sequencing. In one of the inventions of the present invention, immobilization may also refer to the fact that nucleic acids resting or attached to the chip may be detached from the surface of the chip under specified conditions during subsequent reactions such as nucleic acid amplification and/or sequencing. Exemplary non-covalent linkages include, but are not limited to, non-specific interactions (e.g., hydrogen bonding, ionic bonding, van der Waals interactions, etc.) or specific interactions (e.g., affinity interactions, receptor-ligand interactions, antibody-epitope interactions, anti-biotin protein- biotin interactions, streptavidin-biotin interactions, lectin-carbohydrate interactions, etc.). The chip surface linker nucleic acid may also be immobilized on the chip surface by physical adsorption, e.g., by hydrophobic interaction, electrostatic attraction, and the like.

In Step 3 and 4 of the methods of this aspect, the joining of the first barcoded nucleic acid with the chip surface linker nucleic acid, and the joining of the first barcoded nucleic acid with the second barcoded nucleic acid may be carried out by various methods known in the art. For example, the joining is achieved after forming a combination of three nucleic acid fragments (the first barcode nucleic acid, the second barcode nucleic acid, and the linker nucleic acid) under conditions in which a ligation reaction can be carried out, by each being complementary to the sequence of a different end of another single-stranded nucleic acid fragment (the linker nucleic acid).

In one aspect of the present invention, said chip surface linker nucleic acid has at the 5' end a primer fragment for a subsequent amplification reaction, for example a universal primer sequence for use in a known sequencing method.

In one aspect of the invention, said chip surface linker nucleic acid has at the 5' end a motif or sequence for attachment to a chip surface. For example, where the chip surface is modified with aldehyde, the chip surface linker nucleic acid has an amino group at the 5' end. In one aspect of the invention, said chip surface linker nucleic acid has a ligand that can form a specific interaction with a factor modified on the chip, for example said factor and ligand are antibody-epitope, anti-biotin protein-biotin, streptavidin-biotin, lectin-carbohydrate, respectively.

In one aspect of the present invention, said first barcode nucleic acid comprises a first barcode fragment.

In one aspect of the present invention, said second barcode nucleic acid comprises a second barcode fragment. In one aspect of the present invention, said second barcode nucleic acid has a capture fragment at the 3' end for recognizing and binding to a target in a biological sample, such as a fragment for recognizing and binding to mRNA or cDNA, such as a poly-T sequence for recognizing mRNA.

In one of the aspects of the present invention, the 3' end of said first barcode nucleic acid has a first linker fragment for linking to a second barcode nucleic acid. In another one of these aspects of the present invention, said 5' end of said second barcode nucleic acid has a second linkage fragment for linkage to the first barcode nucleic acid. In yet another one of these aspects of the present invention, said first linker fragment and second linker fragment each form a complement to one end of the linker nucleic acid, and under conditions facilitating ligation reaction (e.g., in the presence of a T4 ligase, etc.), the first linker fragment and the second linker fragment form a combination with the linker nucleic acid to achieve the ligation of the first barcode nucleic acid and the second barcode nucleic acid.

The microchips prepared by the method provided by the present invention can be used for the analysis of molecules contained in cells, including nucleic acids and proteins, on tissue samples, in particular thin sections of tissues, e.g., by PCR, mass spectrometry, next-generation sequencing, or ELISA, to obtain their expression and spatial information.

The use of the microchips of the present invention, comprise contacting a tissue section with the chips on which probes recognize and bind nucleic acids, particularly mRNA, from cells in the tissue. Subsequent analyses include reverse transcription and amplification, among others, and may be performed by high-throughput next-generation sequencing (NGS) or synthetic sequencing (SBS).

The method for preparing a chip having a probe array provided by the present invention enables parallel synthesis of a plurality of chips having arrays of identical coding regions on the same substrate. A plurality of sets of identical first bar code strips and second bar code strips can be formed in a first direction and a second direction of the chip substrate as desired, thereby obtaining a plurality of chips having probes defined by identical first bar code and second bar code sequences at corresponding array points.

WO/2022/135598 discloses a method for preparing a biochip having an array in which a first set of barcoded nucleic acids is immobilized directly onto an optically epoxy-modified glass chip by means of a plurality of parallel-set microfluidic devices. In both laboratory and large-scale industrial production environments, the inventors have found that when attempting to combine nucleic acid molecules with modified chips using microfluidic channels, the hydrodynamic properties of the microfluidic channels impose extremely high requirements on the conditions of the reaction between the nucleic acids and the modified groups on the surface of the chip occurring within the channels, including the reaction temperature, the concentration of the reactant, and the ambient humidity and pressure. During the reaction process, if fluctuations in the reaction conditions occur, the results of the reaction between the nucleic acids and the modifying groups on the chip surface will be greatly affected, resulting in serious quality control defects of the product, including defects such as a large area of diminished probe signals on the chip or even the appearance of "null spot" on the chip (no probe signals on the chip).

The inventor unexpectedly found that, in the improved preparation process provided by the present invention, when fixing the nucleic acid of said first barcode nucleic acids on the surface of the chip, the efficiency of fixing can be significantly improved by linking the nucleic acid of said first set of barcode nucleic acids with nucleic acids (referred to herein as chip surface linker nucleic acids) that have been fixed on the surface of the chip (the entire surface of the chip or sections of the surface of the chip having the same position as the first set of barcode nucleic acids) through a nucleic acid-to-nucleic acid linkage reaction (e.g., with a nucleic acid ligase and a nucleic acid linker fragments bridging the two nucleic acids to be linked), which resulting in a significant increase in the density of probes at the array's dots and a significant improvement in uniformity of the probes at the array's dots. Without being limited by this theory, the inventors believe that this is due to the fact that the efficiency and stability of the nucleic acid-to-nucleic acid linkage reaction is stronger than that of other ways of immobilizing nucleic acids on the surface of the chip (e.g., covalent bonding ways, etc.), increasing the efficiency of linkage of the individual fragments, etc. In addition, said chip surface linker nucleic acid can be shorter in length, which leads to be more efficiently immobilized on the chip by means of chemical bonding, etc., and binds in greater numbers. This significantly contributes to the improvements in the dimensional homogeneity of the probe array spots of the prepared chips, the amount of probes that the probe array dotes have, and the homogeneity of the amount of probes (which is reflected in the prepared chips as the intensity of the fluorescent signals carried by the probes as detected by fluorescence microscope and camera, and is reflected in the applications as the number of genes and transcripts identified in the samples, such as in the tissues and the cells, and so on).

The present invention also provides a biochip for analyzing nucleic acid information of biological samples. In one aspect of the present invention, said biochip for analyzing nucleic acid information of a biological sample is prepared by the aforementioned method. In one aspect of the present invention, said chip for analyzing nucleic acid information of a biological sample has a surface having probes forming an array, said array comprising orthogonal rows and columns of probes, said probes in said array each having a different sequence that can be used to indicate the spatial location of said probes. In one aspect of the present invention, said probes comprise a first barcode and a second barcode. In yet another one of these aspects of the present invention, each row of probes of said array of probes has the same first barcode and each column of probes has the same second barcode; each row of probes has a different first barcode and each column of probes has a different second barcode from the other row or column. In one aspect of the present invention, said chip for analyzing nucleic acid information of a biological sample has chip surface linker nucleic acids on its entire surface. In yet another one of these aspects of the present invention, the 5' end of each probe in said probe array is said chip surface linker nucleic acid. In yet another one of these aspects of the present invention, each probe in said probe array comprises, from the 5' end to the 3' end, said chip surface linker nucleic acid, a first barcode, a second barcode, and a capture fragment for recognizing and binding to a target nucleic acid in a biological sample. In yet another one of these aspects of the present invention, each probe in said probe array includes a primer fragment at the 5' end for an amplification reaction. In yet another one of these aspects of the present invention, the sequence of each probe in said probe array further comprises a unique molecular identifier (UMI).

The microarrays provided by the present invention can be used for the analysis of tissue samples, in particular tissue thin sections, for the analysis of molecules contained in cells, including the analysis of nucleic acids and proteins, for example by PCR, mass spectrometry, next-generation sequencing, or ELISA, to obtain expression and spatial information thereof.

The present invention also provides methods for analyzing spatial transcriptomics information of a biological tissue sample, said methods comprising contacting an array of the aforementioned chip with a tissue sample. A "tissue sample" for the purposes of the present invention includes tissue obtained from a subject, fixed, sectioned and mounted on a flat surface. The tissue sample may be, for example, a formalin-fixed paraffin-embedded (FFPE) tissue sample or a fresh tissue sample or a frozen tissue sample. The methods of the present invention may be performed before or after staining the tissue samples. For example, after hematoxylin and eosin staining, the tissue sample may be used for spatial transcriptomics analyzed according to the methods provided herein. The methods may include analyzing the histology of the sample (e.g., using hematoxylin and eosin staining) and then spatially analyzing the tissue. Tissue sections fixed in formalin and embedded in paraffin (FFPE) typically include fixing tissue obtained from a subject in formaldehyde (e.g., 3%-5% formaldehyde in phosphate-buffered saline) or Bouin solution, embedding it in wax, cutting it into thin sections, and then mounting it on a flat surface, such as a microscope slide for biopsy. The method of the present invention involves contacting the tissue section with a probe array on a microchip, which recognizes and binds nucleic acids, particularly mRNA, from the cells in the tissue. Subsequent analyses include reverse transcription and amplification, among others, and can be performed by high-throughput next-generation sequencing (NGS) or synthetic sequencing (SBS).

"Sequencing" generally refers to methods and techniques for determining the sequence of nucleotide bases in one or more polynucleotides. A polynucleotide may be, for example, a nucleic acid molecule such as deoxyribonucleic acid (DNA) or ribonucleic acid (RNA), including variants or derivatives thereof (e.g., single-stranded DNA). Sequencing may be performed by various systems currently available, such as, but not limited to, sequencing systems through Illumina, Pacific Biosciences, Oxford Nanopore, or Life Technologies. Alternatively or additionally, sequencing may be performed using nucleic acid amplification, polymerase chain reaction (PCR) (e.g., digital PCR, quantitative PCR, or real-time PCR), or isothermal amplification. Such systems may provide a plurality of raw genetic data corresponding to genetic information of a subject (e.g., a human), such as samples provided from the subject generated by the system. In some examples, such systems provide a sequencing read length (also referred to herein as a "read length"). The read length may include a string of nucleic acid bases that correspond to the sequence of the sequenced nucleic acid molecule. In some embodiments, the systems and methods provided herein may be used in conjunction with proteomic information.

In some embodiments, nucleic acids from tissue sections (e.g., formalin-fixed paraffin-embedded (FFPE) tissue sections) are transferred to the array and captured onto the array by hybridization to a capture probe. In some embodiments, the capture probe may be a universal capture probe that hybridizes, for example, to an articulator region in a nucleic acid sequencing library, or to a poly-A tail of mRNA. In some embodiments, the capture probe may be a gene-specific capture probe that hybridizes, for example, to an mRNA or cDNA specifically targeted in the sample.

In some embodiments, nucleic acids from tissue sections (e.g., FFPE sections) are transferred to the array and captured on the array by single-stranded ligation to a universal articulator oligonucleotide. In other embodiments, nucleic acids on a chip may be transferred to a tissue section (e.g., an FFPE section). Methods known in the art may be used to enable probes bound to the chip to enter cells on the tissue with which they are in contact after being dislodged in solution. For example, a photolyzable connector or the like may be added to the binding of the nucleic acid probe to the chip, or the nucleic acid probe may be bound to the chip by a pH-sensitive connector, and then the nucleic acid probe may be detached from the chip by changing the pH of the solution.

With the chip of the present invention and the use thereof, the spatial position of the probe molecule in the array on the surface of the chip can be known from the first barcode sequence and the second barcode sequence of the probe molecule that each array dot has, and thereby information on the position in said tissue of the cell in which the nucleic acid molecule is contained can be obtained.

In one aspect of the present invention, said method further comprises subjecting thin sections of said tissue to morphological analysis and/or histological analysis, which is performed by H&E staining, IHC staining, ISH staining, and FISH staining.

In one aspect of the present invention, one or more biomolecules in the tissue sample may be analyzed in said method, which may be performed, for example, by PCR, mass spectrometry, next generation sequencing, or ELISA.

In one aspect of the present invention, the tissue sample in said method is from an animal, a farm animal, a pet, a human subject.

In one aspect of the present invention, the biomolecule in said method comprises one or more of a non-human cell, a human cell, an unnatural protein, a nucleic acid, or a small molecule, a dye, a virus, a bacterium, a parasite, a protozoan, or a chemical substance.

In one aspect of the present invention, the small molecule in said method comprises a semi-antigen, a peptide tag, a protein tag, a fluorescent tag, a nucleic acid tag, and combinations thereof.

In one aspect of the present invention, the analysis in said method comprises generating quantitative and/or qualitative data for the marker.

In one aspect of the present invention, the marker in said method comprises DNA, proteins, RNA, lipids, organelles, metabolites, or cells.

In one aspect of the present invention, the marker in said method comprises a genomic polymorphism, a pharmacogenomic single nucleotide polymorphism (SNP), a genomic SNP, a somatic polymorphism, and differential expression of a protein, a lipid, and/or an organelle.

In one aspect of the present invention, the marker in said method comprises an altered nucleotide sequence coding for an altered amino acid sequence, a chromosomal translocation, an intrachromosomal inversion, a copy number change, an expression level change, a protein level change, a protein activity change, or a methylation status change in a cancerous tissue or a cancerous cell, as compared to a normal healthy tissue or cell.

In one aspect of the present invention, the marker is measured in said method by single-cell sequencing, single-nucleus sequencing, flow cytometry, immunohistochemical staining, hematoxylin and eosin staining, whole-genome sequencing, high-throughput sequencing, mass spectrometry, DNA microarray, or a combination thereof.

In one aspect of the present invention, said method further comprises subjecting thin sections of said tissue to morphological analysis and/or histological analysis, which histological analysis is performed by H&E staining, IHC staining, ISH staining, and FISH staining.

In one aspect of the present invention, the analysis of the one or more biomolecules in said method is performed by PCR, mass spectrometry, next generation sequencing, or ELISA.

### Example 2 Preparation of the biochip

FIG. 1 shows a flow chart of an exemplary embodiment of a method for preparing a biochip provided by the present invention.

In this embodiment, a glass slide is used as the chip substrate, and the surface of the chip is modified with reactive groups such as amino groups, aldehyde groups, epoxy groups, isothiocyanate groups, sulfhydryl groups, silanes, and other reactive groups by a surface chemical reaction.

In this Example, a commercially-available glass sheet modified with epoxy groups (Nexterion^{®} Slide E) is used as the chip substrate.

One hundred 5' end amino modified Set I barcoded nucleic acids with the following sequences were synthesized:

5'    amino-CTACACGACGCTCTTCCGATCT    12345678
CTCTTTCCCTACACGACGCTCTT- 3'

Synthesize one 5' end amino-modified universal chip surface linker nucleic acid having the following sequences, where the underlined T base is FITC modified:
5' amino- CTACACGACGCTCTTCCGATC- 3'

Synthesize 100 5' end phosphorylation-modified Set I barcoded nucleic acids with the following sequences:
5' phosphorylated-CTCTTTCCCTAC12345678 ACGACGCTCTTC- 3'.
wherein "12345678" denotes a barcode fragment having 8 nucleotides, wherein the sequence of said 8 nucleotides is known (specified). The sequence of said barcode fragment (referred to as the first barcode) of each of said 100 first barcode nucleic acids are different from each other, and the sequence of the first barcode of each first group barcode nucleic acid is known (specified). wherein the underlined T base is FITC modified. In this embodiment, fluorescence modification of the barcode fragments and detection of the resulting fluorescence signal is used for observation or production quality control of each step of incorporation of the barcode fragments in the chip synthesis. In other embodiments, fluorescence modification of the barcode fragment may not be present.

Synthesize 100 Set II of barcode nucleic acids having the following sequences: 
5’ phosphorylated -GAGTGATTGCTTGTGACGCCTT 87654321
NNNNNNNNNN TTTTTTTTTTTTTTTTTTTTTTTTTTTTTTVN- 3'
 wherein "87654321" denotes a barcode fragment having 8 nucleotides, wherein the sequence of said 8 nucleotides is known (specified), wherein the sequences of the barcode fragments (referred to as second barcodes) of said 100 Set II barcode nucleic acids are different from each other, and wherein the sequence of the second barcodes of each Set II barcode nucleic acid is known (specified). The underlined T base is Cy3 modified.

Synthesize one first linker nucleic acid with the following sequence:
5'- AGGGAAAGAGAGATCGGAAG - 3'

Synthesize one second linker nucleic acid with the following sequence:
5'- GCAATCACTCGAAGAGCGT - 3'.

Fit the cell culture chamber to the slide and use a frame to press the chamber to the slide to improve sealing. Use a pipette to add 10-20 uM of the universal chip surface linker nucleic acid (dissolved in 300 mM sodium phosphate buffer pH 8.5) into the chamber, spreading all over the bottom surface of the chamber, and then place the slides in a thermostatic mixer at 40°C, 800 rpm for 3 hrs of mixing reaction. At the end of the reaction, the modified slides were washed with 0.1% Triton X-100, 1 mM HCl, 100 mM KCl, and then blocked at 50 °C using 0.1 M Tris pH 9.0, 50 mM ethanolamine, and 0.1 % SDS. The substrate was rinsed using deionized water for 1 min after the end of blocking, and then the substrate was blown dry with nitrogen. Fluorescence microscopy was used to observe the FITC fluorescence signal of the universal nucleic acid to determine the completion of the reaction and the effect of surface modification.

A device including a plurality of microfluidic channels disposed in parallel with openings at the bottom of the microfluidic channels is prepared by a soft lithography process made of polydimethylsiloxane (PDMS) as shown in FIG. 2.

The device of the microfluidic channels includes about 50-500 parallel set microfluidic channels. The width of each of said parallel provided microfluidic channels is about 2-200µm, preferably about 5-50µm, most preferably about 5-25µm, for example, about 5µm, 10µm, or 50µm. the spacing between each of the adjacent microfluidic channels is about 5-400µm, preferably about 10-100µm, most preferably about 10-50µm, for example, about 20µm, 50µm or 100µm.

The PDMS microfluidic was fixed to the slide to provide a flow channel. A clamping tool can be used to press the microfluidic device to the substrate slide to improve the sealing. One end of the microfluidic channel is the solution inlet and the other end is connected to the vacuum suction device through an interface.

The PDMS microfluidic device was fitted to the slide, passed through buffer to remove air in the channels, and rinsed once more with buffer, and then 10-20µM of the first set of barcode nucleic acids was added to the flow channel: one type of the first barcode nucleic acid and the first linker nucleic acid and T4 ligase were passed through each flow channel (the first barcode nucleic acid of each flow channel has a different barcode sequence from the first barcode nucleic acid of the other flow channels). After filling the flow channels, the slides were left to stand for 30 min at 37°C. After the reaction was completed, the substrate was rinsed with deionized water for 1 min, and then the substrate was blown dry with nitrogen. A fluorescence microscope was used to observe the FITC fluorescence signal of the first barcode nucleic acids to determine the completion of the reaction between the first set of barcode nucleic acids and the first set of barcode nucleic acids, i.e., the fixation of the first set of barcode nucleic acids on the chip surface which were covered by the channels to form the first set of barcode bands.

Another PDMS microfluidic device is fixed to the slide along a direction that forms an orthogonal direction to the flow channel of the first PDMS microfluidic device. After passing buffer into the flow channel to expel gas from the flow channel, 10-20µM of the second set of barcode nucleic acids was passed: one second barcode nucleic acid (the second barcode nucleic acid of each flow channel has a different barcode sequence from the second barcode nucleic acid of the other flow channels) and the second linker nucleic acid and T4 ligase were passed into each flow channel. The flow channels were filled and left to stand at 37°C for 30 min.

After completion of the ligation reaction, 1× PBS buffer was passed through and the runners were cleaned with ultrapure water. After that, the device was removed and the substrate was rinsed with deionized water for 1 min, and then the substrate was blown dry with nitrogen. A fluorescence microscope was used to observe the Cy3 fluorescence signal of the second barcode nucleic acid to determine the completion of the reaction and the effect of linkage reaction between the second barcode nucleic acid and the first set of barcode nucleic acids at the intersection of the flow channel to form the barcode array, and thus complete the chip preparation.

The chips obtained from the preparation were vacuum encapsulated and stored at room temperature or 4°C in a refrigerator protected from light. The efficiency of the ligation reaction between each barcode nucleic acid as well as the intensity (density) and uniformity of the probes of the prepared chip were evaluated by observing the fluorescence signals on the prepared chip.

FIG. 3 shows a diagram of the detection of Cy3 fluorescence signals on the obtained chip carried by the second set of barcoded nucleic acids. Photographs and measurements were taken by a fluorescence microscope (Olympus IX53) and a CCD camera (Olympus DP74).

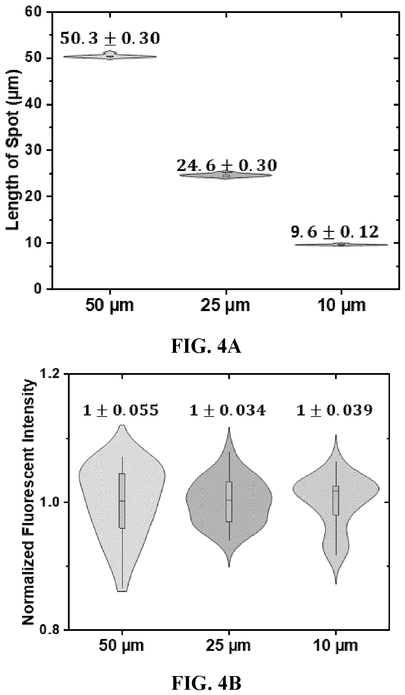

FIG. 4A and FIG. 4B show the results of detection of probes on a chip obtained by microfluidic channel preparation with widths of 50µm, 25µm, and 10µm(i.e., probe dots of sizes of 50µm × 50µm, 25µm × 25µm, and 10µm × 10µm, respectively), respectively, according to the method described in Example 2. The images obtained by taking pictures and measurements through a fluorescence microscope (Olympus IX53) and a CCD camera (Olympus DP74) were analyzed, and the fluorescence intensity values and boundaries were extracted by ImageJ to calculate the dot array intensity homogeneity and the actual width of the dot array.

FIG. 4A and FIG. 4B show the results of the measurements of the dimensions and fluorescence intensity of the dots of the array, respectively. The results of FIG. 4A show that the deviation of the dimensional homogeneity of each probe dot on the chip prepared by the present invention was less than 0.6% (50µm), 1.2% (25µm) and 1.3% (10µm), respectively. The results of Fig. 4B show that the homogeneity deviations of the probe are less than 5.5 % (50µm), 3.4% (25µm) and 3.9% (10µm), respectively. It can be seen that the chips prepared by the present invention have a very high uniformity of dot size, as well as the uniformity of the modified probes on each dot.

### Example 3 Tissue sample preparation and staining

### (I) Tissue OCT embedding

Collect fresh mouse brain tissue samples, rinse the tissue surface residue with pre-cooled PBS solution or saline, and then absorb the liquid with clean absorbent paper. Place the tissue in the embedding tank and add OCT embedding agent until the tissue is completely covered. Make sure there are no air bubbles around the tissue and place the embedding tank on dry ice until the OCT is completely frozen.

### (ii) Frozen Sections

Set the temperature of the frozen sectioning machine to -20° C for the chamber and -10° C for the sample head. Before sectioning, the frozen tissues and substrates were first equilibrated in the -20°C freezer sectioning machine chamber for more than 30 minutes, and then frozen sectioning was performed in the freezer sectioning machine chamber with a thickness of 10µm.

### (iii) Tissue fixation and HE staining

Attach the cut tissue sections to the barcode array-modified substrate prepared in Example 2, and then incubate at 37°C for 1 minute. Completely immerse the adhered tissue substrate in pre-cooled methanol and fix it at -20°C for 30 minutes. At the end of fixation, the substrate was removed, the liquid on the backside was wiped dry, and 500µl of isopropanol was added dropwise to the tissue sections and incubated for 1 min at room temperature. After 1 min, the isopropanol was removed and then air-dried for 5-10 min at room temperature.

Add 1 ml of hematoxylin to evenly cover the tissue sections on the substrate and incubate for 7 minutes at room temperature. Remove hematoxylin reagent, wash the substrate by immersing it in RNase-free Water and air dry. Add 1 ml of bluing solution and incubate for 2 minutes at room temperature. Remove the bluing solution, wash the substrate by immersing it in RNase-free Water and dry the liquid on the back of the substrate. Add 1 ml of Eosin mixture and incubate for 1 minute at room temperature.

Remove the eosin, wash the substrate by immersing it in RNase-free Water and air dry until the tissue is opaque. After incubating the slides for 5 min at 37°C, bright field imaging was performed.

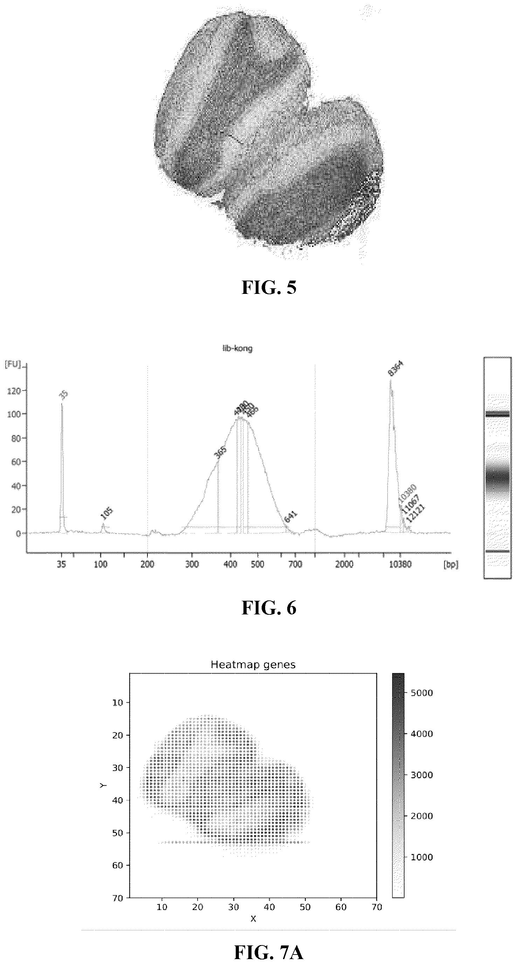

FIG. 5 shows the tissue images of the mouse brain tissue after HE staining.

### (iv) Tissue permeabilization

Assemble a fixture chamber onto the tissue chip, making sure that each of the tissue sections are located inside the corresponding chambers. Add 70ul of permeabilizing enzyme (0.1% pepsin diluted in 0.1N HCl) to the chamber to permeabilize the tissue at 37°C, remove the permeabilizing enzyme and then wash with 0.1× SSC.

### (v) Chip quality inspection

The chip prepared in Example 2 was taken out from a -20°C refrigerator after storage for 6 months and 12 months, respectively, to which the reaction solutions of the aforementioned steps (iii) tissue fixation, HE staining and (iv) tissue permeabilization were added, and after treatment under the said reaction conditions, the intensity (density) and homogeneity of the probe of the chip were tested in accordance with the method as documented in Example 2.

### Example 4 Reverse transcription reaction of tissue sample sections

Add 70 µl of reverse transcription mix to the chamber in Example 3. The reverse transcription mix comprises: 1x first strand buffer, 5mM DTT, 500µM dNTP, 0.19µg/µl BSA, 1% DMSO, 2.5µM Template Switch Oligo, 20U/µl Superscript III, and 2U/µl RNase inhibitor.

The Template Switch Oligo sequence was:
5'Biotin-AAGCAGTGGTATCAACGCAGAGTACATrGrGrG-3'

In the Template Switch Oligo, the penultimate 1, 2 and 3 bases were modified with riboguanine nucleosides.

After sealing the chamber with adhesive tape, the reaction mixture was placed on a temperature-controlled plate and set to approximately 50°C for reverse transcription for 16 hours.

At the end of reverse transcription, aspirate the reverse transcription mixture from the chamber. The chamber was incubated for 5 min at room temperature with 70 µl of 0.08 M KOH, and then washed once with 100 ul of RNase-free water.

Add cDNA second strand synthesis reaction solution to the cleaned chamber. The second strand synthesis reaction solution included 1× First Strand Buffer, 10U Klenow Exo⁻, and 2.5µM Second Strand Primer. After sealing the chamber with tape, the chamber was placed on a temperature-controlled plate and set to about 37°C for cDNA second strand synthesis for 1 hour.

The Second Strand Primer sequence is:
5'-AAGCAGTGGTATCAACGCAGAGTACAT-3'

At the end of the reaction, the second strand synthesis reaction mixture inside the chamber was aspirated and then washed once by adding 100ul of RNase-free Water. Next, 35 µl of 0.08 M KOH was added to the chamber and incubated for 10 min at room temperature. Prepare several new 1.5 ml centrifuge tubes by adding 10 µl Tris (1 M, pH 7.0). Transfer 35 µl of sample from the chamber to the corresponding Tris-containing centrifuge tubes and mix well to complete the preparation of second-strand of cDNA.

### Amplification of cDNA

A new 1.5 ml centrifuge tube was placed on ice to prepare the PCR amplification reaction solution. The PCR reaction solution consisted of 1×Kapa HiFi Hotstart Ready Mix, 0.8µM cDNA Forward Primer, 0.8µM cDNA Reverse Primer, and 35µl cDNA template, in a total volume of 100µl. cDNA amplification was carried out by the following protocol:

| | | |
|---|---|---|
| 98°C | 3min | 1 |
| 98°C | 15s | **15 circles** |
| 63°C | 20s | |
| 72°C | 1min | |

Where the cDNA Forward Primer sequence was:
5'-CTACACGACGCTCTCTTCCGATC - 3'

The cDNA Reverse Primer sequence is:
5'-AAGCAGTGGTATCAACGCAGAG - 3'

After amplification, the amplified product was purified using 0.6 × AMpure XP Beads.

The amplified product was subjected to library building/sequencing.

### Example 5 Library construction and sequencing

### Fragmentation, end repair, poly A tailing

Take a new PCR tube and place it on ice to prepare the fragmentation reaction solution. The fragmentation reaction solution included: 5 µl of FEA Buffer V2, 10 µl of DNA purified in the previous step, 25 µl of ddH₂O, and 10 µl of FEA Enzyme Mix V2, in a total volume of 50 µl. Blow or shake the mixing solution using a pipette and centrifuge it briefly to collect the reaction solution at the bottom of the tube. Place the PCR tube in the PCR instrument and run the following program:

| Heat start 105°C | On |
|---|---|
| 37°C | 20 min |
| 65°C | 30 min |
| 4°C | Hold |

This results in the fragmentation of the DNA while blunting the ends of the fragmented DNA and phosphorylating the 5' end and adding the dA tail at the 3' end.

### Adapter Ligation

A new PCR tube was taken and placed on ice and the adaptor ligation reaction solution was prepared. The adaptor reaction solution consists of 25 µl of Rapid Ligation Buffer V2, 50µl of the fragmented DNA purified in the previous step, 15 µl of ddH₂O, 5µl of Rapid DNA Ligase V2, 5µl of the adaptor (10 pM), in a total volume of 100 µl. The reaction solution is blown and shaken using a pipette, and the reaction solution is collected at the bottom of the tube by brief centrifugation. Place the PCR tube in the PCR instrument and run the following program:

| Heat start 105°C | On |
|---|---|
| 20°C | 15 min |
| 4°C | Hold |

Wherein the adaptor is:
5'phosphorylated-GATCGGAAGAGCACACGTCTGAACTCCAGTCA*C-3'
5'-GCTCTTCCGATC*T-3'

The last base in the adaptor is modified by thiolation.

At the end of the ligation reaction, purify the ligation product using 0.6× XP SPRIselect Beads.

### Library amplification

A new PCR tube was placed on ice to prepare the library amplification reaction solution. The library amplification reaction solution consisted of 25 µl of VAHTS HiFi Amplification Mix, 20µl of DNA ligated with the adaptor purified in the previous step, and 5 µl of Index PCR Primer Mix (10 pM each), in a total volume of 50 µl. The reaction solution was blown and shaken with a pipette, and centrifuged briefly to collect the reaction solution at the bottom of the tube. Place the PCR tube in the PCR instrument and run the following program:

| Heat start 105°C | On | |
|---|---|---|
| 95°C | 3 min | |
| 98°C | 20 s | |
| 63°C | 30 s | 13 circles |
| 72°C | 20 s | |
| 72°C | 1 min | |
| 4°C | Hold | |

Where the Index PCR Primer sequence is:
i5 index primer: 5'-AATGATACGGCGACCACCGAGATCTACAC-[i5 index]-ACACTCTTTCCCTACACGACGCTC-3'
i7 index primer:

5'- CAAGCAGAAGACGGCATACGAGAT - [i7 index]
-GTGACTGGAGTTCAGACGTGT-3'

After the amplification reaction, the amplification products were purified using 0.9× AMpure XP Beads.

### Library quality control

The constructed libraries were tested for concentration and length distribution using Qubit and Agilent Bioanalyzer High Sensitivity chip, respectively. As shown in FIG. 6, the concentration of the library measured by Qubit was no less than 20 ng/µl, and the length distribution test was performed by taking 1µl of the library sample and following the instructions of the instrument and the kit to perform the fragment distribution test, and the fragments of the library is distributed in the range of 200-600 bp.

### Sequencing

PE150 sequencing of the library was performed using illumina NovaSeq 6000.

### Data processing and analysis

a) Collect UMI and Barcode data from Read1 using data processing software such as umitools (version: 1.1.2).
b) Match read2 to the mouse reference genome Mouse reference, mm10 (GENCODE vM23/Ensembl 98) by using data processing software such as STAR (version: 2.5.3a)
   featureCounts (Version 2.0.3) assign the gene.
c) Generate expression matrices using data processing software such as umitools (version: 1.1.2).
d) Compare the Barcode analysis with HE images using Adobe illustrator.

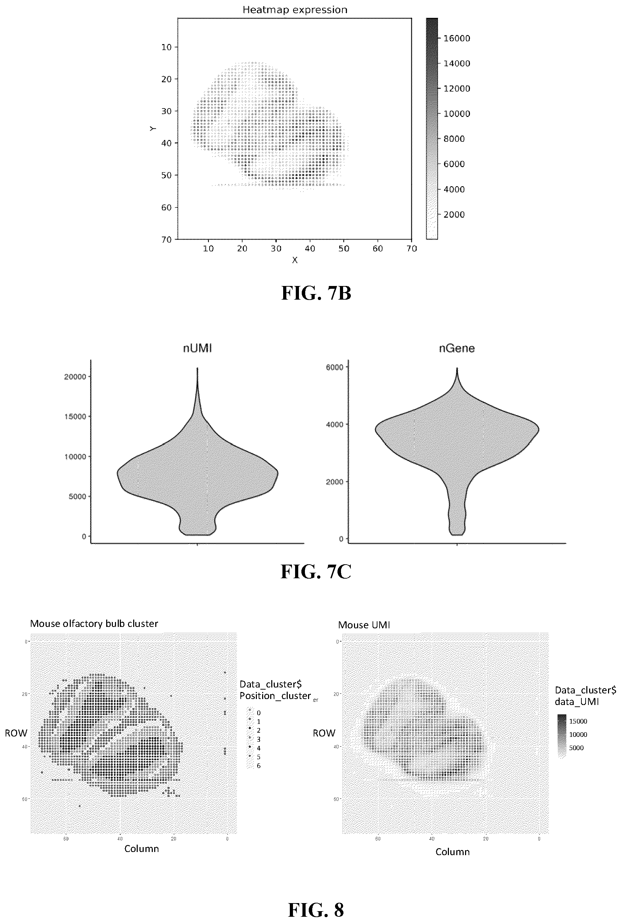

The results of transcriptome analysis of mouse olfactory bulb are shown in FIG. 7. FIG. 7A shows a graph of the number of genes detectable by spatial transcriptomics analysis of mouse brain tissue with the biochip provided by the present invention. FIG. 7B shows a graph of the number of UMIs detectable by spatial transcriptomics analysis of mouse brain tissue with the biochip provided by the present invention. FIG. 7C shows a graph of the distribution of the actual number of genes detected versus the number of captured transcripts (UMI) by spatial transcriptomics of mouse brain tissue with the biochip provided by the present invention.

The obtained data are subjected to unsupervised cluster analysis, and the results are shown in FIG. 8.

The foregoing is only a preferred embodiment of the present invention and is not intended to limit the present invention, and any modifications, equivalent substitutions, improvements, etc., within the spirit and principles of the present invention shall be included in the scope of protection of the present invention.

## Claims

1. A method for preparing a biochip having an array, comprising the steps of:
Step 1. providing a chip;
Step 2. fixing a chip surface linker nucleic acid on the chip surface;
Step 3. applying a first set of barcode nucleic acids on the surface of the chip through a plurality of microfluidic channels set in parallel to form a plurality of first barcode strips in a first direction under the condition of enabling a joining reaction between said chip surface linker nucleic acid and the first barcode nucleic acids, wherein said first set of barcode nucleic acids comprise a plurality of first barcode nucleic acids having different barcode sequences, each said first barcode strip contains one kind of first barcode nucleic acid, and each said kind of first barcode nucleic acid fixed in each first barcode strip has a different barcode sequence;
Step 4. applying a second set of barcode nucleic acids in a second direction on the chip surface through a plurality of microfluidic channels set in parallel to form a plurality of second barcode strips, said second set of barcode nucleic acids comprise a plurality of second barcode nucleic acids having different barcode sequences, each said second barcode strip contains one kind of second barcode nucleic acid, and each said kind of second barcode nucleic acid contained in each second barcode strip has a different barcode sequence;
Step 5. under conditions that cause the first barcode nucleic acid and the second barcode nucleic acid to undergo a joining reaction, joining the second barcode nucleic acid to the first barcode nucleic acid on the surface of the chip where said plurality of first barcode strips intersect said plurality of second barcode strips to generate probes, said probes forming arrays of dots, each dot containing a probe with a position-specific sequence.

2. The method of claim 1, wherein said first set of barcode nucleic acids or second set of barcode nucleic acids are delivered and fixed to the chip surface using a microfluidic device having a plurality of microfluidic channels set in parallel, wherein the side of said microfluidic channel in contact with the chip surface is set to allow passage of solution or nucleic acids in solution.

3. The method of claim 2, wherein to each of channels of said microfluidic device, a barcode nucleic acid of said first set of barcode nucleic acids or said second set of barcode nucleic acids having different barcode sequences is added, preferably, wherein the sequence of the barcode fragment of each of said first barcode nucleic acids of said first set of barcode nucleic acids and the sequence of the barcode fragment of each of said second barcode nucleic acids of said second set of barcode nucleic acids is designated.

4. The method of claim 1, wherein said second barcode nucleic acid in said second set of barcode nucleic acids comprises a probe fragment at the 3' end for recognizing and binding a target nucleic acid in the biological sample (e.g., a fragment for recognizing and binding mRNA or cDNA, e.g., a poly-T sequence) and a second barcode fragment, preferably, said second barcoded nucleic acid in said second set of barcoded nucleic acids further has a unique molecular identifier (UMI).

5. The method of claim 1, wherein said chip surface linker nucleic acid has a linking fragment at the 3' end for linking to the first barcode nucleic acid via a first single-stranded linking nucleic acid, and said first barcode nucleic acid has a linking fragment at the 5' end for linking to the chip surface linker nucleic acid via said first single-stranded linking nucleic acid, said linking fragment at the 3' end of chip surface linker nucleic acid and said linking fragment at the 5' end of the first barcode nucleic acid are each complementary to a sequence at each end of said first single-stranded linking nucleic acid.

6. The method of claim 1, wherein said first barcode nucleic acid has a first linking fragment at the 3' end for linking to the second barcode nucleic acid via a second single-stranded linking nucleic acid, and said second barcode nucleic acid has a second linking fragment at the 5' end for linking to the first barcode nucleic acid via said second single-stranded linking nucleic acid, said first linking fragment and second linking fragment are each complementary to a sequence at each end of said single-stranded linking nucleic acid.

7. The method of claim 1, wherein said chip surface linker nucleic acid is fixed on the chip surface by chemical bonding means, said chemical bonding means being any chemical group reactions, for example, amino-aldehyde group reaction, a covalent cross-linking and the like.

8. The method of any one of claims 1-7, wherein in array dots of the chip, the probes have a density of about 10³-10⁵ probes/µm².

9. The method of any one of claims 1-7, wherein in array dots of the chip, the probes have a homogeneity deviation of less than 20%, preferably less than 10%, more preferably less than 6%.

10. The method of any one of claims 1-7, wherein in array dots of the chip, the probes have a deviation in dimensional homogeneity of less than 10%, preferably less than 5%, more preferably less than 2%.

11. A biochip for analyzing nucleic acid information of a biological sample, wherein said biochip has a surface having probes forming an array thereon, said array comprising orthogonal rows and columns of probes, the probe in each spot of said array having a different code sequence, wherein said probes comprise a first barcode and a second barcode, each row of probes of said array of probes has the same first barcode and each column of probes has the same second barcode; each row of probes has a first barcode different from the other row and each column of probes has a second barcode different from the other column; preferably, there are a chip surface linker nucleic acid on the whole surface of said biochip.

12. The biochip of claim 11, wherein each probe in said probe array comprises, from the 5' end to the 3' end, said chip surface linker nucleic acid, a first barcode, a second barcode, and a capture fragment for recognizing and binding to a target nucleic acid in a biological sample.

13. The biochip of claim 11, wherein in array dots of the chip, the probes have a density of about 10³-10⁵ probes/µm².

14. The biochip of claim 11, wherein in array dots of the chip, the probes have a homogeneity deviation of less than 20%, preferably less than 10%, more preferably less than 6%.

15. The biochip of any one of claims 11-14, which is prepared by the method of any one of claims 1-10.

16. A method for analyzing spatial transcriptomic information of a biological tissue sample by using a biochip having an array of any one of claims 11-15, said method comprises: contacting the array of the biochip with the tissue sample, the probes on the array recognizing and binding nucleic acids, in particular mRNA, of cells in the tissue.

17. The method of Claim 16, which further comprising the step of releasing nucleic acids from the cells of the tissue for contacting with the probes on said biochip, preferably, when said tissue is a fixed and embedded tissue, the method further comprising the step of releasing the nucleic acids from the cells by permeabilizing said tissue;
optionally, the method further comprising performing a reverse transcription reaction;
optionally, the method further comprising amplifying said cDNA molecule;
optionally, the method further comprising performing library building/sequencing of the amplified obtained nucleic acids.

18. The method of claim 17, wherein said biological sample is a tissue sample from a subject, for example a surgically excised tissue sample, preferably a thin section of tissue obtained by microtome processing.

19. The method of claim 18, wherein further comprising subjecting said thin section of tissue to morphological analysis and/or histological analysis, the histological analysis being performed by H&E staining, IHC staining, ISH staining, and FISH staining.
